# EUROPEAN PATENT SPECIFICATION

(11) **EP 4 388 976 B1**
(45) Date of publication and mention of the grant of the patent: **20.08.2025**
(21) Application number: 22215444.5
(22) Date of filing: 21.12.2022
(51) Int. Cl.: A61B 1/05, A61B 1/00

(54) **ENDOSCOPE WITH NOISE MITIGATION**
ENDOSKOP MIT RAUSCHMINDERUNG
ENDOSCOPE AVEC ATTÉNUATION DU BRUIT

(43) Date of publication of application: 26.06.2024
(73) Proprietor: Ambu A/S, 2750 Ballerup (DK)
(72) Inventor: NAIR, Kamalath Swathi Narayanan, 2750 Ballerup (DK); NIROUMAND, Farideh Javidi, 2450 Copenhagen SV (DK); OHLSSON, Jonas Mathias, 261 61 Landskrona (SE)
(74) Representative: AWA Denmark A/S

(56) References cited:
- WO-A1-2022/050663
- JP-A- H10 234 661
- US-A1- 2012 292 079

## Description

### TECHNICAL FIELD

The present disclosure relates to intrusive medical devices comprising distal end cameras, such as endoscopes and respiratory tubes, and, in particular, intrusive medical devices configured to mitigate electrical noise.

### BACKGROUND

Visualization systems including video processing apparatus electrically connected to an intrusive medical device are known and are can be used for visual navigation into, and examination and diagnosis of, hollow organs and body cavities, as well as, optionally, to assist in surgery, e.g. for a targeted tissue sampling. Example intrusive medical devices include endoscopes and respiratory tubes. Endoscopes include procedure-specialized endoscopes, such as bronchoscopes, arthroscopes, cystoscopes, ureteroscopes, cholangioscopes, colonoscopes, laparoscopes, gastroscopes, and duodenoscopes. Respiratory tubes include endobronchial tubes, endotracheal tubes, tracheostomy tubes, and other tubes configured to ventilate at least a portion of the respiratory system or lungs of a patient, and may include a laryngeal mask or inflatable cuffs. A visualization system including an endoscope operable with a surgical tool is described in commonly-owned U.S. Patent No. 10,464,107. Respiratory tubes are described in commonly-owned U.S. Patents Nos. 9,486,595, 9,889,264, 10,406,309, and 10,888,679. A visualization system including a portable medical monitor having a display screen is described in commonly-owned U.S. Patents Nos. 11,266,297 and 11,328,390. Document US 2012/292079 A1 describes a signal cable for an endoscope, the signal cable electrically connecting an image pickup section and a subsequent stage signal processing section of the endoscope. The signal cable consists of a bundle of at least four conductors twisted at a pitch of 13 to 15mm.

An electrosurgical instrument may be guided through a lumen of the intrusive medical device to perform medical procedures within the patient's body cavity. Known electrosurgical tools, which are operated by high voltage pulses (e.g. in a range of 2 kV to 8 kV), may generate high frequency noise in signals and interference in videos apparent to a user during a procedure. An electrosurgical tool, which is configured to perform argon plasma coagulation (APC), is an example of such electrosurgical tool. Argon plasma coagulation is an electrosurgical, monopolar procedure for superficial hemostasis, devitalization and ablation using ionized argon gas, which as an inert gas can be easily ionized. High voltage pulses result in a strong electric field (high frequency) that may be experienced as a high frequency noise on electrical conductors. Electrical noise in video signals can also be caused by high camera clock speeds.

For single-use intrusive medical devices, it is important that the entire device be manufactured economically and inexpensively. Therefore, components of single-use intrusive medical devices are mainly made of polymeric materials to enhance disposability, reduce the size, and reduce costs.

Electrical noise can result in the loss of live images for various reasons, including "freezing" of the camera module (requiring a reset) and because the communication of configuration of control data to the camera goes wrong, i.e. data is written in wrong places, or the wrong data is written, in registers of the camera.

Furthermore, known intrusive medical devices may comprise diameters greater than 3.4 mm and might have enough space to not impose size or configuration limits on the electrical conductors. However, as the intrusive medical devices become smaller in size, particularly when their distal ends are 3.4 mm or less in diameter, which is desirable to mitigate tissue damage and facilitate navigation into the patient, it is desirable to reduce the size and configuration of the bundle.

Even further, it is desirable to present images on the display screen in real-time. Known medical monitors may employ image processing algorithms configured to mitigate the effects of electrical noise. For example, medical monitors might average frames of a video stream (e.g. a sequence of frames or images) or exclude frames from the video stream if the frames are defective, e.g. exhibit vertical or horizontal lines, exhibit large over or under-exposed areas, or are defective for other reasons. This may cause a physician to see a frame that is outdated, albeit by a fraction of a second, which is not ideal. If image processing algorithms are modified to present video streams at or closer to real-time, these video stream noise mitigation techniques are removed and the electrical noise in the intrusive medical devices has to be reduced or eliminated to ensure an adequate user experience.

Additionally, image processing algorithms may be modified to add features, such as navigation and tissue/object identification, which if added without removal of code may require larger, more expensive, hardware. Thus, removal of noise mitigation processing instructions may be enabled by improvements in the intrusive medical devices, resulting in overall cost reductions.

For at least the foregoing reasons, it is desirable to incorporate noise mitigation features in intrusive medical devices, particularly in single-use medical devices, to improve their value and, in particular, to improve their effectiveness when noise generating tools are used.

### BRIEF DESCRIPTION OF THE DISCLOSURE

The objectives of the present disclosure are to provide an intrusive medical device with features that eliminate or at least reduce the disadvantages of the prior art intrusive medical devices and suitably deal with the above-described problems. In particular, it is an object of the present disclosure to present an intrusive medical device that exhibits reduced electrical noise relative to prior art intrusive medical devices.

The objectives of the present disclosure are satisfied by an intrusive medical device in accordance with claim 1 and by a visualization system in accordance with claim 15. Advantageous embodiments are claimed in the dependent claims and/or are explained below.

In one embodiment according to the first aspect, an intrusive medical device comprises a proximal end and a distal end spaced apart from the proximal end; a tubular member defining a lumen therein, the tubular member extending from the proximal end to the distal end; a camera positioned at the distal end; and a bundle of at least four unpaired conductors, the bundle extending from the proximal end to the distal end and electrically connected to the camera at the distal end, wherein the bundle is twisted at a pitch of 15 mm +/- 10 mm.

In one variation according to the present embodiment, the at least four unpaired conductors include a ground conductor, a camera power conductor, a clock conductor and a data conductor, wherein the at least four unpaired conductors are arranged cross-sectionally with the data conductor positioned between two of the at least four unpaired conductors, and wherein the two of the at least four unpaired conductors does not include the clock conductor.

In one example of the present variation, the at least four unpaired conductors consist of five unpaired conductors including the ground conductor, the camera power conductor, the clock conductor, the data conductor, and a light power conductor, and the data conductor is positioned immediately next to the ground conductor and/or the camera power conductor and/or the light power conductor.

In some examples of the present embodiment, each of the conductors is insulated, and each of the conductors, without the insulation, comprises a diameter of between 0.0790 and 0.210 mm.

In some examples of the present embodiment, at least three of the at least four conductors are coaxial.

In one variation of the present embodiment, the intrusive medical device comprises an endoscope including a positioning interface having a distal end; an insertion cord connected to and extending from the distal end of the positioning interface and comprising an insertion tube, a bending section, and a distal tip, the camera positioned in the distal tip, the tubular member extending from the positioning interface through the insertion tube to the distal tip, wherein the bundle extends from the positioning interface to the distal tip.

In one example according to the present variation, the bundle of at least four unpaired conductors is enclosed in an electrical shield. The distal tip may comprise a diameter smaller than 3.4 mm.

In one example according to the present variation, the bundle of at least four unpaired conductors is enclosed in an electrical shield and the electrical shield is electrically disconnected at the distal end of the bundle. The distal tip may comprise a diameter smaller than 3.4 mm.

In one example according to the present variation, the bundle of at least four unpaired conductors is enclosed in an electrical shield, the bundle has a proximal end, and the electrical shield is only grounded at the proximal end of the bundle. The distal tip may comprise a diameter smaller than 3.4 mm.

In further examples according to the present variation, the distal tip comprises a diameter smaller than 3.4 mm.

In further examples according to the present variation, the distal tip comprises a diameter smaller than 3.4 mm, the bundle of at least four unpaired conductors is enclosed in an electrical shield, and the electrical shield is electrically disconnected at the distal end of the bundle.

In another variation according to the present embodiment, the intrusive medical device comprises a tubular member having a circumferential wall defining a lumen therein; and an illumination lumen within the circumferential wall, wherein the camera and the bundle of at least four unpaired conductors are positioned in the illumination lumen.

In one example according to the present variation, the intrusive medical device comprises an inflatable cuff positioned at the distal end. The intrusive medical devise may comprise a dual-lumen tube comprising a medial wall dividing the lumen into a first lumen and a second lumen. The at least four unpaired conductors may include a ground conductor, a camera power conductor, a clock conductor and a data conductor, the at least four unpaired conductors are arranged cross-sectionally with the data conductor positioned between two of the at least four unpaired conductors, and the two of the at least four unpaired conductors does not include the clock conductor. The at least four unpaired conductors may consist of five unpaired conductors including the ground conductor, the camera power conductor, the clock conductor, the data conductor, and a light power conductor, and the data conductor may be positioned immediately next to the ground conductor and/or the camera power conductor and/or the light power conductor. At least three of the at least four conductors are coaxial.

In one example according to the present variation, the at least four unpaired conductors may include eight conductors including two pairs of twisted pair conductors. The at least four unpaired conductors may include eight conductors including a video out conductor positioned in a center of the bundle and surrounded by the other of the eight conductors.

In an embodiment according to the second aspect, a visualization system comprises an intrusive medical device according to the first aspect and any of the variations and examples thereof, and a video processing apparatus configured to communicatively connect with the intrusive medical device to receive a video stream therefrom.

A person skilled in the art will appreciate that any one or more of the above aspects of this disclosure and embodiments thereof may be combined with any one or more of the other aspects of this disclosure and embodiments thereof.

### BRIEF DESCRIPTION OF THE DRAWINGS

Embodiments of this disclosure will be described in more detail in the following with regard to the accompanying figures. The figures are not to be construed as limiting other possible embodiments falling within the scope of the attached claim set.
FIGS. 1 and 2 are schematic illustrations of an intrusive medical device according with the detailed description;
FIG. 3 is a schematic illustration of an electrosurgery system and a visualization system including an intrusive medical device;
FIGS. 4 and 5 are perspective views of an embodiment of an intrusive medical device, exemplified by an endoscope;
FIGS. 6 to 10 are depictions of cross-sections of embodiments of bending sections of endoscopes including the endoscope depicted in FIGS. 4 and 5;
FIGS. 11 and 12 are plan and sectional views of another embodiment of an intrusive medical device, exemplified by a dual-lumen tube;
FIGS. 13 and 14 are perspective and plan views of embodiments of video processing apparatus operable with the intrusive medical devices of FIGS. 1 to 12;
FIGS. 15 and 16 are cross-section and schematic views of an embodiment of a bundle of unpaired conductors according with the detailed description;
FIG. 17 is a cross-section view of a variation of the embodiment of the bundle of unpaired conductors of FIG. 15;
FIG. 18 is a cross-section view of another variation of the embodiment of the bundle of unpaired conductors of FIG. 15;
FIG. 19 is a schematic view of a further variation of of the embodiment of the bundle of unpaired conductors of FIG. 15; and
FIGS. 20 and 21 are cross-section and schematic views of a further embodiment of a bundle of unpaired conductors according with the detailed description.

### DETAILED DESCRIPTION

The term "distal," as used herein, refers to a direction or position that is generally towards a target site, and the term "proximal," as used herein, refers to a direction or position that is generally away from the target site.

Unless otherwise defined, all technical and scientific terms used herein have the same meaning as commonly understood by one of ordinary skill in the art. In case of conflict, the present document, including definitions, will control. Preferred apparatuses and materials are illustrated below, although apparatuses, and materials similar to those illustrated herein may be used in practice or testing. The materials, and examples disclosed herein are illustrative only and not intended to be limiting.

As described herein, an intrusive medical device may comprise a proximal end and a distal end spaced apart from the proximal end; a camera positioned at the distal end; and a bundle of at least four unpaired conductors, the bundle extending from the proximal end to the distal end and electrically connected to the camera at the distal end, the at least four unpaired conductors including a ground conductor, a camera power conductor, a clock conductor and a data conductor, the bundle twisted at a pitch of 15 mm +/- 10 mm. The intrusive medical device may comprise an endoscope.

An intrusive medical device may comprise a proximal end and a distal end spaced apart from the proximal end; a tubular member defining a lumen therein, the tubular member extending from the proximal end to the distal end; a camera positioned at the distal end; and a bundle of at least four unpaired conductors, the bundle extending from the proximal end to the distal end and electrically connected to the camera at the distal end, the at least four unpaired conductors including a ground conductor, a camera power conductor, a clock conductor and a data conductor, the bundle twisted at a pitch of 15 mm +/- 10 mm.

Advantageously, whether with or without a tubular member defining a lumen therein, the intrusive medical device as described above mitigates electrical noise. The electrical noise might be cross-talk and/or generated by an electro-surgical tool (EST) or other electromagnetic field, while reducing the cost of a bundle as compared to use of paired twisted wires or coaxial cables. The data conductor may transfer digital or analog video data. The data conductor may also transfer control signals in addition to video signals.

The at least four unpaired conductors may be arranged cross-sectionally with the data conductor positioned between two of the at least four unpaired conductors, where the two of the at least four unpaired conductors do not include the clock conductor.

The bundle of at least four unpaired conductors may be enclosed in an electrical shield. The electrical shield may be electrically disconnected at the distal end of the bundle. The bundle has a proximal end. The electrical shield may only be grounded at the proximal end of the bundle.

Having described intrusive medical devices generally, attention is now directed to more detailed descriptions of embodiments of said intrusive medical devices.

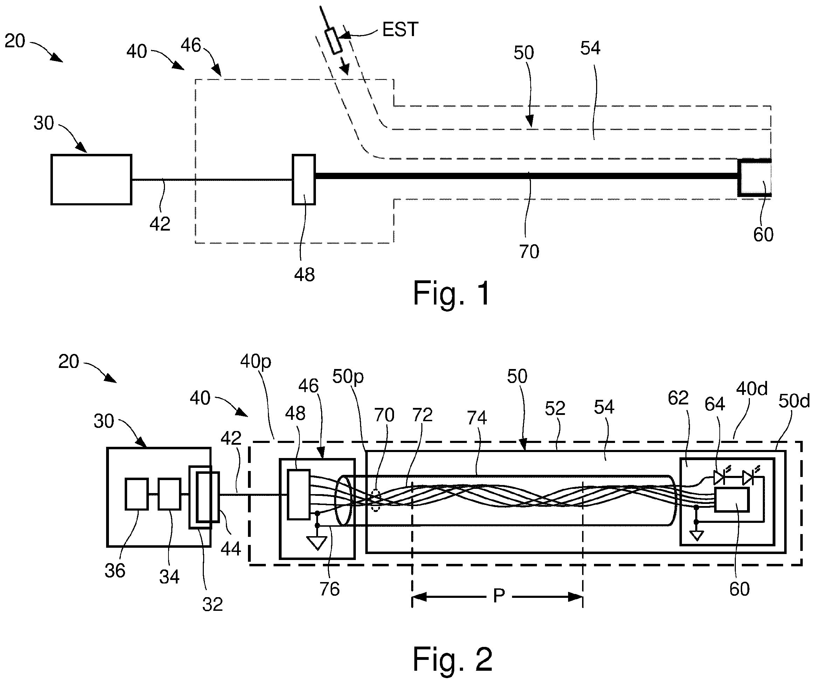

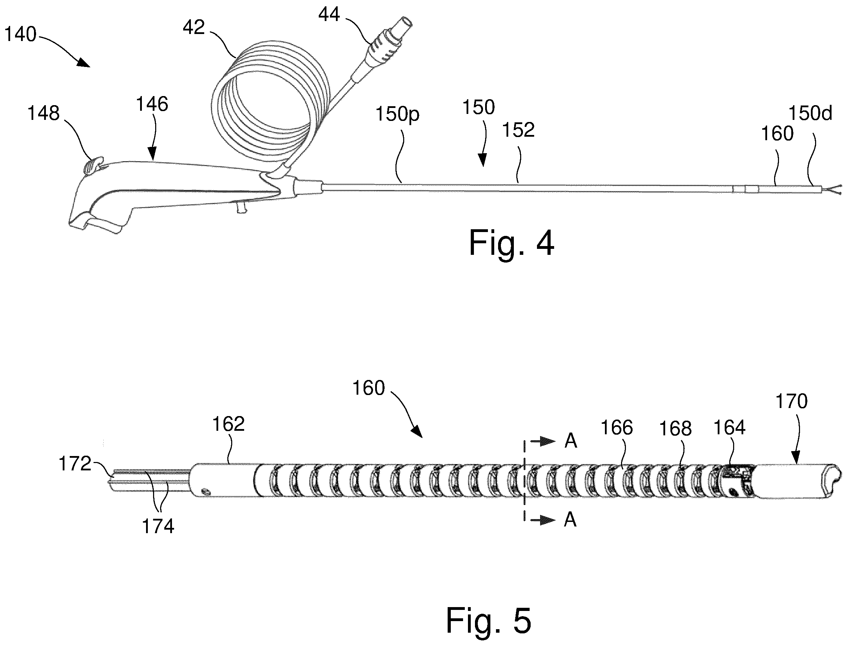

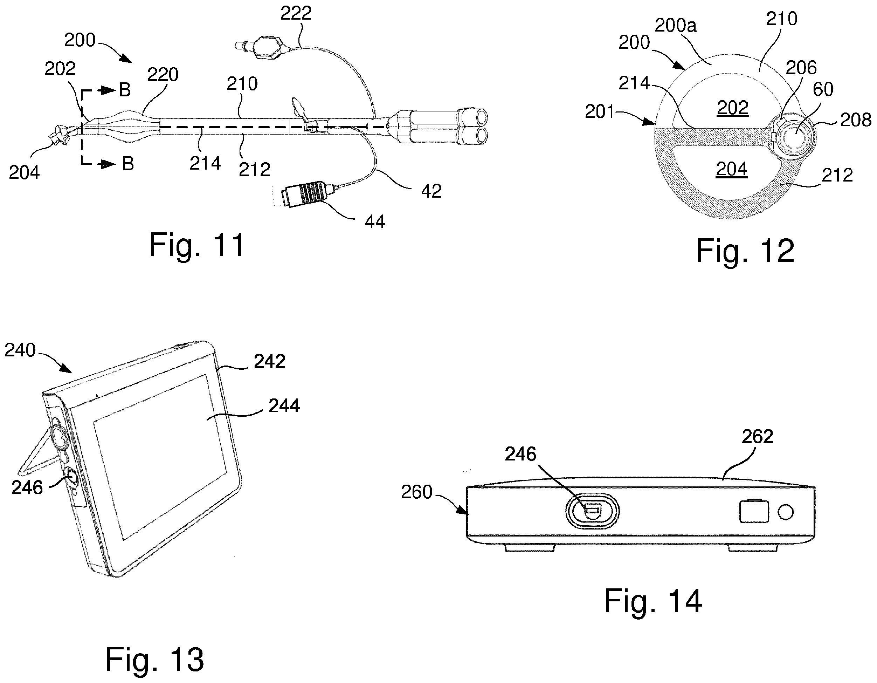

Figs. 1 and 2 are schematic diagrams of an embodiment of a visualization system 20 including a video processing apparatus 30 electrically connected to an intrusive medical device 40 by a cable 42 and a cable connector 44. Examples of the intrusive medical device 40 include endoscopes (described with reference to FIGS. 4 to 10), dual-lumen tubes (described with reference to FIGS. 11 and 12), and any other device configured for insertion into the body of a patient, human or animal, and containing illumination means at a distal end thereof. An electrosurgical tool EST useable with the visualization system 20 is also shown. Some of the features of the intrusive medical device 40 shown in FIGS. 1 and 2 illustrate optional features and components. Embodiments of illumination means include light emitting diodes (LEDs) and fiber lightguides extending from the proximal end 40p to the distal end 40d and connected, at the proximal end, to an illumination source such as an LED.

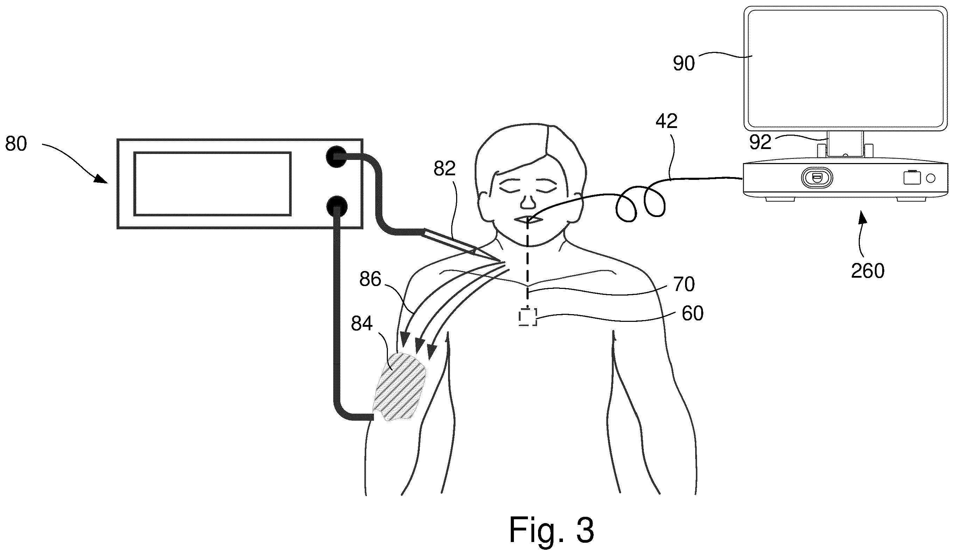

As shown in FIG. 2 and described in detail further below, the video processing apparatus 30 comprises a cable connector receptor 32, an input circuit 34, and a processor 36. Optionally, the VPA 30 may include a housing supporting a display screen connected to the processor 36 and operable to present images provided by the processor 36. A VPA with a display screen is described with reference to FIG. 13. A VPA without display screen is described with reference to FIGS. 3 and 14. The input circuit 34 may comprise a deserializer circuit to convert the data or signals provided by an image sensor of a camera from serial to parallel format. The processor 36 may comprise an FPGA, CPU, GPU, and combinations thereof. An FPGA may be programmed to conduct video processing to improve the images and a CPU may be provided to configure a graphical user interface (GUI) and superimpose the GUI onto the images. The combined content may be supplied to the FPGA, which may be connected to a video output circuit.

The intrusive medical device 40 has a proximal end 40p and a distal end 40d spaced apart from the proximal end 40p and comprises the cable 42 and the cable connector 44, an optional positioning interface 46, an optional circuit board 48, a tubular member 50 having a proximal end 50p, a distal end 50d, and a tubular member wall 52 defining a lumen 54 configured to receive therethrough the EST. The tubular member 50 extends from the proximal end 40p to the distal end 40d.

The intrusive medical device 40 also comprises a camera 60, an optional circuit board 62, optional light emitting diodes (LED) 64, a bundle 70 of electrical conductors, an optional electrical shield 74, and an optional proximal-end shield ground 76 (e.g. a connection of the electrical shield 74 to ground). The camera may include an image sensor, lenses and a lens support coupled with the image sensor. The image sensor may have a cross-section of less than 2.0 mm on each side, preferrably less than 1.9 mm on each side. The bundle 70 of electrical conductors extends from the distal end 50d to at least the proximal end 50p. The bundle 70 comprises at least four unpaired conductors 72 connected to the camera 60 at the distal end 50d. In some embodiments, the at least four unpaired conductors 72 include a ground conductor, a camera power conductor, a clock conductor and a data conductor. The bundle 70 is twisted at a pitch P. In some embodiments, the pitch P is in a range of 15 mm +/- 10 mm. In some embodiments, the pitch P is in a range of 5 mm to 15 mm.

As described with reference to FIGS. 1 and 2, the intrusive medical device may comprise a tubular member defining a lumen extending from the proximal end to the distal end. However, the intrusive medical device may be devoid of such a lumen. For example, some endoscopes are used for inspection and can be introduced into the patient through a lumen of another intrusive medical device that has one or more lumens. The advantages of the electrical noise mitigation features described herein are equally applicable to such an endoscope because electrosurgery may be conducted with different tools and systems. In some systems the EST is introduced through the lumen of the intrusive medical device 40. In some systems the EST is not introduced through the lumen of the intrusive medical device 40.

FIG. 3 illustrates an example of an electrosurgery system 80 comprising two electrodes, 82 and 84, that form an electrical path 86 via the patient's body. The EST, e.g. electrode 82, may be introduced into the patient through a lumen of a tubular member (not shown). A separate bundle 70 connected to the camera 60 and the cable 42 is shown to illustrate that the bundle 70 does not necessarily form part of an intrusive medical device having the tubular member through which the EST is introduced into the patient. The cable 42 is connected to a VPA 260, which is described with reference to FIG. 14 and includes a separable display screen 90 and a display screen support 92, which is configured, in normal use, to be detachable from the VPA 260 and the display screen 90. The display screen is operable to present images and video streams generated and transmitted by the camera 60. A VPA 240 (described below) may be used instead of the VPA 260.

FIGS. 4 and 5 show an embodiment of an intrusive medical device 40, exemplified by an endoscope 140, comprising a positional interface exemplified by a handle 146, which includes a steering control 148 operable to steer the distal end of the endoscope 140, as is known in the art, by alternativelly pulling on steering cables 174, shown in FIG. 5, responsive to movement of the steering control 148. The endoscope 140 comprises an insertion cord 150 having a proximal end 150p and a distal end 150d, the insertion cord 150 including an insertion tube 152 and a bending section 160. A distal tip comprising a tip housing 170 extends from the bending section 160. The camera 60 is positioned in the tip housing 170. The camera 60 may at least partly be positioned in the tip housing 170. The bending section 160 may comprise a single-piece polymeric structure comprising a plurality of segments 166 intermediate a proximal segment 162 and a distal segment 164 that is connected to the tip housing 170. The segments are interconnected by polymeric strips 168, or hinges, which form part of the one-piece structure and bend upon tensioning of the steering cables 174. A working channel tube 172, which is an example of the tubular member 50, provides the lumen 54 for introduction of the EST.

Instead of a single-piece polymeric structure, the bending section can also be assembled from multiple pieces. Such assemblies may be formed from two single-piece polymeric structures that elongate and form two, opposing, longitudinal halves of the finished bending section. Such assemblies may also be formed from individual segments assembled via hinges.

The endoscope 140 may be a single-use device. Single-use devices are designed to be low cost and disposable, not to be cleaned and sterilized after use and not to be used after cleaning.

A positioning interface functions to control the position of the insertion cord. A handle is an example of a positioning interface and, unless stated otherwise, the terms are used interchangeably. The handle also functions to provide the steering control, e.g. knobs, levers, buttons, and the like, to steer the field of view of the camera. Alternatively, a different positioning interface can be provided that is connected to the insertion cord and is detachably connected to a robotic arm. The insertion cord thus extends from the robotic arm, and the intrusive medical device is detachable from the robotic arm. The robotic arm responds to signals, including voice commands from an operator, to rotate, translate, and otherwise position the proximal end of the insertion cord, as an operator would do manually. The positioning interface can include control actuators, including manual control actuators. Alternatively or additionally, control actuators can be provided in or on the robotic arm or by the robotic system including the robotic arm, thereby potentially reducing the cost of the intrusive medical device. Example control actuators include single axis actuators, including linear motion actuators. A linear motion actuator may comprise a threaded rod coupled to a threaded nut portion, in which a motor rotates the rod to translate the nut portion.

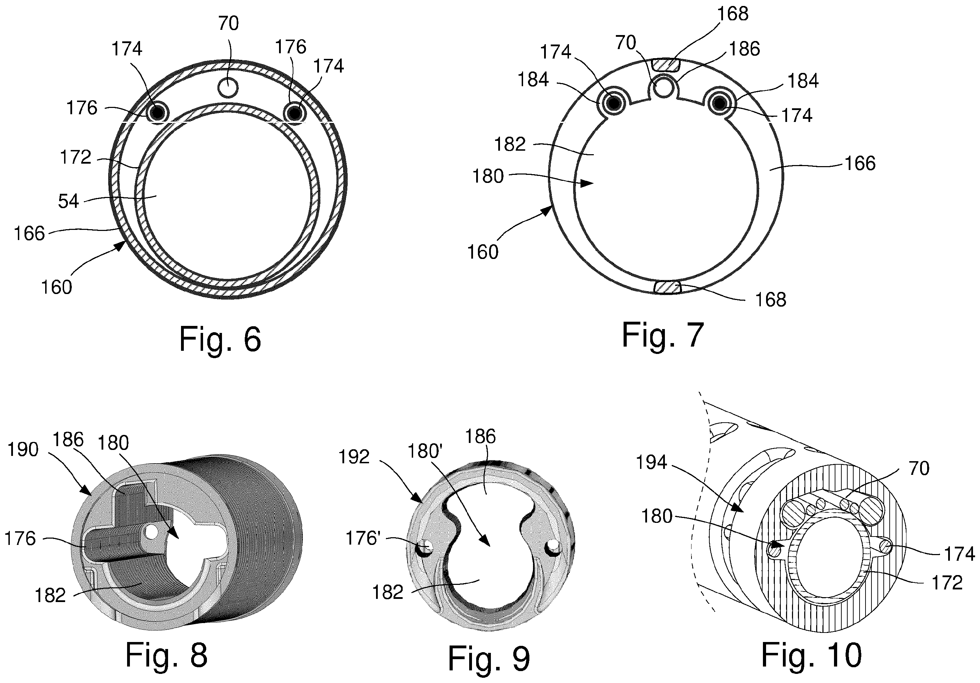

FIG. 6 is a schematic view of a cross-section A-A of the bending section 160 showing a cross-section of one of the segments, a cross-section of the working channel tube 172, the lumen 54, or working channel lumen, the steering cables 174, steering cable guide tubes 176, and the bundle 70 of electrical conductors 72. The steering cable guide tubes 176 surround the steering cables 174, which are connected to the tip housing 170 at one end and to the steering control 148 at the opposite end. A wall of the segment 166 (not shown in FIG. 6) comprises cut-outs or apertures through which the steering cable guide tubes 176 pass. Examples of the cut-outs or apertures are shown and described with reference to FIGS. 7 to 9. A sleeve (not shown) may be provided over the bending section 160 to fluidly seal the spaces between adjacent segments 166. The outer diameter of a segment 166 may be substantially similar or the same as the outer diameter of the tip housing 170. In some embodiments the outer diameter is less than 4.2 mm, less than 3.6 mm, less than 3.4 mm, less than 3.2 mm, and even less than 3.0 mm.

In a variation A of the present embodiment, the wall thickness of the wall of the working channel tube is between, and including, 0.10 and 0.20 mm, preferrably between, and including, 0.12 and 0.18 mm, and more preferrably about 0.15 mm, and the external diameter of the working channel tube is between, and including, 2.0 and 3.0 mm, preferrably between, and including, 2.20 and 2.80 mm, and more preferrably between, and including, 2.40 and 2.60 mm.

In a variation B of the present embodiment, a diameter of the camera cable, which includes the conductors and may comprise the shield, is between, and including, 0.40 and 0.56 mm, preferrably between, and including, 0.44 and 0.52 mm, and more preferrably between, and including, 0.45 mm, and the external diameter of the working channel tube is between, and including, 2.0 and 3.0 mm, preferrably between, and including, 2.20 and 2.80 mm, and more preferrably between, and including, 2.40 and 2.60 mm.

In a variation C of the present embodiment, an internal diameter of the insertion tube is less than 3.8 mm, or less than 3.4 mm, or less than 3.0 mm.

Variations of the present embodiment may be combined to form additional variations of the embodiment. Thus, variation A may be combined in a new variation with variation B, variation A may be combined in a new variation with variation C, variation A may be combined in a new variation with variations B and C, and variation B may be combined in a new variation with variation C.

FIGS. 7 to 10 provide examples of segment walls provided to maintain separation of the steering cables 174 and secure the bundle 70 in the bending section 160. FIG. 7, for example, shows a common opening 180 defining a working channel tube aperture 182, steering cable cut-outs 184 (cut out from the periphery of the working channel tube aperture 182), and a bundle cut-out 186. Providing the cut-outs around the working channel tube aperture 182 allows for reduction of the diameter and increased flexibility of the bending section 160. FIG. 8 shows another example of the opening 180 in a bending section 190. The opening 180 in this example includes, as in FIG. 7, three cut-outs for the steering cables 174 and the bundle 70. Additional tubes may be provided in the cut-out 186 due to its relatively larger cross-sectional area relative to the cross-section of the bundle 70. FIG. 9 shows an example of a common opening 180' in a bending section 192. The opening 180' in this example includes, as in FIG. 7, a cut-out for the the bundle 70, and perhaps for other tubes or components, and apertures 176' for steering cables 174. Additional tubes may be provided in the cut-out 186 due to its larger, relative to the cross-section of the bundle 70, cross-sectional area. FIG. 10 shows an example of the common opening 180 in a bending section 194. The bending section 194 differs from the bending section 192 in that cut-outs are provided for the steering cables 174 instead of openings. As used herein, common opening refers to an opening that accomodates a working channel tube and one or more of steering cables and a bundle. As shown, accomodation of the steering cables and bundle is provided by cut-outs. Openings for the working channel tube, steering cables and the bundle can also be provided, size permitting, via individual openings instead of a common opening.

The camera 60 may have a cross-section of less than 2.0 mm on each side. The outside diameter of the tip housing 170 may be about 3.0 mm, and preferrably about 2.8 mm, and more preferrably 2.8 mm or less. The term "about" is intended to define a range of +/- 10% from the specified numeral.

As the dimensions of the camera and the bending section are continuously reduced for the benefit of the patient, the size of the wires and cables increase as a percentage of the cross-section. It is valuable, to continue reductions in size and cost, to identify cable configurations, the cables comprising the wires, shielding, jacket, etc., that use smaller wires while still avoid the negative effects of electrical noise. The success of the mitigation effort is dependent on the image sensor and deserializer used, the length of the bundle, and the structure surrounding it.

FIGS. 11 and 12 show another embodiment of an intrusive medical device 40, exemplified by a dual-lumen tube 200. FIG. 12 shows a cross-section B-B of the tube 200. The dual-lumen tube 200 comprises a tubular member 200a having a peripheral (or circumferential) wall 201 defining a first lumen 202 and an illumination lumen 208 therein. The camera 60 and LEDs 206 are positioned in the illumination lumen 208 located in the wall of the tube 200.

The dual-lumen tube can be an endobronchial or an endotracheal tube, for example. The dual-lumen tube 200 may also have a second lumen 204. The peripheral wall 201 may consist of a first portion 210 and a second portion 212 divided by a medial wall 214. An inflatable cuff 220 is provided at the distal end and is connected via an inflation lumen (not shown) to an inflation tube 222 that can be connected to a pump to inflate the cuff 220. As with the endoscope 140, it is desirable to reduce the size of the device and increase its flexibility, for example by reducing the wall thickness of the tube, which requires reductions in the size of the camera 60 and the bundle 70. Although not shown, the bundle 70 is positioned in the illumination lumen 208 and communicativelly connects the camera 60 with the cable connector 44. A single-lumen tube can also include the features described herein, including the first lumen 202, the camera 60, the LEDs 206, and the bundle 70, positioned in the illumination lumen 208 located in a wall of the tube 200.

A VPA 240 with a display screen 244 is shown in FIG. 13. The VPA 240 includes a housing 242 and one or more cable connector receptor 246 configured to receive the cable connector 244. The housing 242 supports and is assembled in one-piece with the display screen 244, referred to as an "integral" display screen, by contrast with a "separable" display screen. The terms "integral" and "separable" reflect a device in its assembled form, as it is in use, by contrast with a device in its unassembled or disassembled form. A VPA 260 without a display screen 244 is shown in FIG. 14 and was previously shown in FIG. 3 with a separable display screen 90. The VPA 260 includes a housing 262 and one or more cable connector receptacles 246 configured to receive the cable connector 44. In both the VPA 240 and the VPA 260, a separable display screen can be communicativelly connected thereto, as is known in the art, via an ethernet, wireless, AVI, HDMI, or other data interfaces. Upon connection of an intrusive medical device 40, the VPA 240 or 260 present images or video streams on the integral and/or the separable display screen, as is known in the art.

In the foregoing description intrusive medical devices were described, the intrusive medical devices comprising: a proximal end and a distal end spaced apart from the proximal end; a camera positioned at the distal end; and a bundle of at least four unpaired conductors, the bundle extending from the proximal end to the distal end and electrically connected to the camera at the distal end, the bundle twisted at a pitch of 15 mm +/- 10 mm. In some embodiments but not others, the intrusive medical devices include a tubular member defining a lumen therein, the tubular member extending from the proximal end to the distal end of the device. In some embodiments but not others, the at least four unpaired conductors include a ground conductor, a camera power conductor, a clock conductor and a data conductor. Embodiments of the bundle 70 are described below.

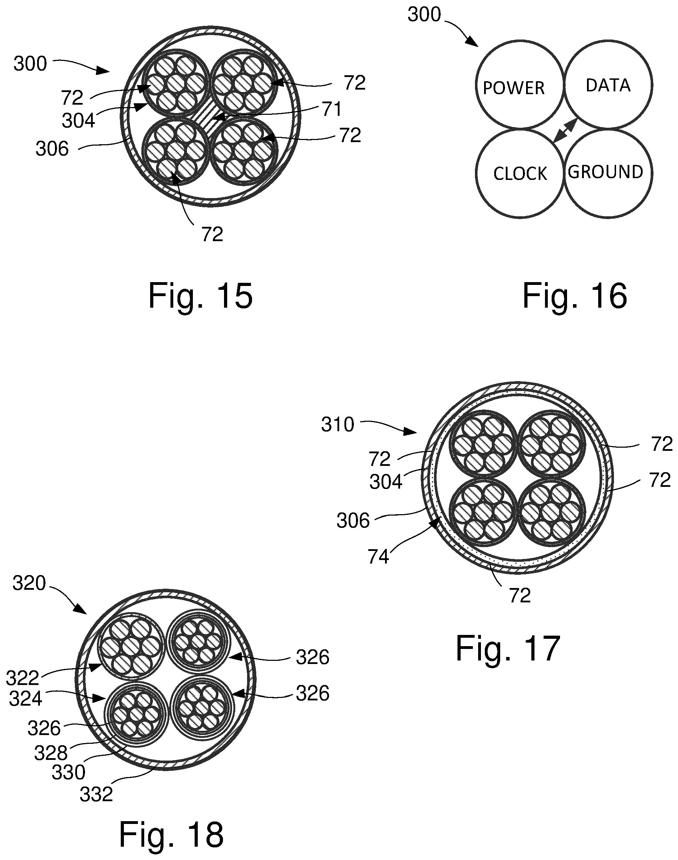

Attention is now directed to an embodiment of the bundle 70, shown in FIGS. 15 and 16, which is denoted by numeral 300. FIG. 16 is a schematic depiction of the position of conductors 72 in the bundle 300. Each conductor may be a wire, illustrated by a stranded wire comprising 7 strands. Each conductor is surrounded by an insulator 304. The bundle 300 includes four wires 72. A filler 71 of standard materials may be included. Each wire might also be a solid wire or a stranded wire with more or less than 7 strands. The insulator might be a plastic sheath or a coated insulator. The wires are selected based on their current carrying capacity and physical strength and therefore may be larger or smaller depending on their function. To reduce size and increase flexibility of the tubular member, it is desirable to use the smallest wire that can perform the selected function. Therefore, one of the wires might be larger than another of the wires. The voltage drop across the length of a wire is another limiting factor in some functions. For example, too much voltage drop, due to too small wire diameter, can cause image degradation in the data conductor of the bundle. The bundle 300 may be enclosed in a protective sleeve, or jacket, 306 that provides strength to the bundle, permitting use of smaller gauge wires, and which also maintains the pitch of the bundle. The inventors have found that particular combinations of wires, wire positions, and pitch result in surprisingly good electrical noise mitigation, for example, but not necessarily exclusivelly, of cross-talk between the clock onto the video signal.

A 4-wire bundle as depicted in FIGS. 15 and 16 is operable with image sensors comprising four connection pads. Such image sensors and pad arrangements have been developed to reduce the size of the image sensor. Reductions in bundle cross-section area are thus beneficial to reduce the cross-section area of the intrusive medical device 40 and benefit from use of smaller cameras. The power conductor in a 4-wire bundle may provide power to both the camera and the LEDs. The LED's may, alternatively, be powered by a 5^{th} conductor.

In a variation A of the present embodiment, the bundle 70 of wires, which is denoted by numeral 310 in FIG. 17, is surrounded by an electrical shield 74 in addition to a jacket 306. The wires are not coaxial wires and thus are not individually shielded.

In a variation B of the present embodiment, some of the wires of the bundle 70, which is denoted by numeral 320 in FIG. 18, are individually shielded. The bundle 320 differs from the bundle 300 in that one of the wires, denoted by numeral 322, is not coaxial and the other wires, denoted by numeral 324, are coaxial and include an insulation 326, an electrical shield 328 surrounding the insulation, and a jacket 330 surrounding the electrical shield. A binder tape 332 is provided to maintain the pitch of the twisted bundle, which may be a 5, 10, 15, 20, or 25 mm pitch or between 5 and 25 mm. The bundle 320 is devoid of an electrical shield surrounding the bundle. If space allows, a jacket can be provided instead of in addition to binder tape. However, because tape is thinner, tape is preferrable as space limitations increase.

In an example of variation B, the wire 324 is larger than the wire 326. In one example, the wire 324 is at least a 42 American Wire Gauge (AWG) wire and may be larger, e.g. a 40 AWG wire, and the wire 326 is at most a 44 AWG wire, preferrably a 46 AWG wire. The bundle 324 together with the binder tape may be referred to as a "image sensor cable". Generally, the term "image sensor cable" refers to the combination of conductors, shield(s), and jackets that are assembled together and extend through the insertion tube as a unit. Wire gauge may also be based on the length of the cable. If the image sensor cable (inside the device) is 300 mm or less a thinner gauge may be used than if the cable is longer.

In another variation, C, of the present embodiment, all the wires are coaxial and the image sensor cable is devoid of an electrical shield surrounding the entire bundle.

In a further variation, D, of the present embodiment, the conductors comprise the same type and size of conductor.

Variations of the present embodiment may be combined to form additional variations of the embodiment. Thus, variations A and B may be combined in new variations with variations C, D, and C and D.

In a further example, the image sensor cable comprises three coaxial insulated cables and a non-coaxial conductor. The non-coaxial conductor may be the LED power conductor. The coaxial cables may be for image sensor power, signal out, and clock/data.

In a yet further example, the image sensor cable comprises three coaxial uninsulated cables and a non-coaxial conductor. The non-coaxial conductor may be the LED power conductor. The shields of the coaxial cables in this example may touch. The advantages of avoiding the insulation is reductions in size and cost.

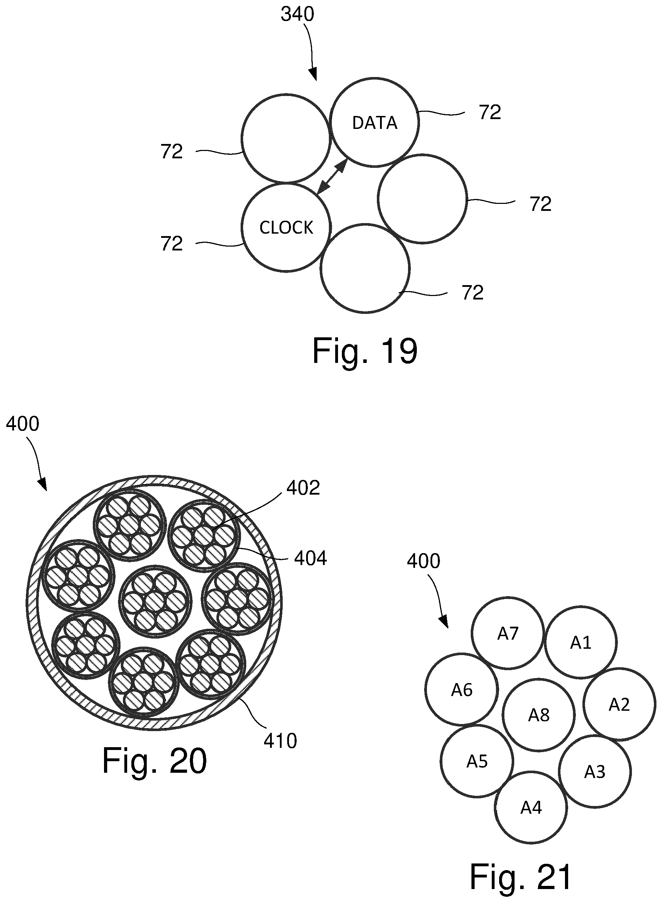

Another embodiment of the bundle 70, which is denoted by numeral 340, will now be described with reference to FIG. 19, which is a schematic depiction of the position of the wires 72. The bundle 340 differs from the bundle 300 in that it includes a separate power wire for the LEDs. Provision of a separate power wire enables control of the LED power via a current source without the provision of a voltage regulator or other power division means to divide the power provided by the camera power wire to the camera and LEDs. Avoidance of power division means at the distal end of the intrusive medical device also enables reduction of the size of the intrusive portion, e.g. the distal end, of the intrusive medical device. In the present embodiment, the at least four unpaired conductors consist of five unpaired conductors including the ground conductor, the camera power conductor, the clock conductor, the data conductor, and a light power conductor, and the data conductor is positioned immediately next to the ground conductor and/or the camera power conductor and/or the light power conductor. In other embodiments, the at least four unpaired conductors may comprise five unpaired conductors including the ground conductor, the camera power conductor, the clock conductor, the data conductor, and a light power conductor, and the data conductor is positioned immediately next to the ground conductor and/or the camera power conductor and/or the light power conductor.

In a variation A of the present embodiment, an electrical shield surrounds the bundle of conductors and a sheath or jacket surrounds the electrical shield. The bundle, electrical shield and jacket may be referred to, collectivelly, as a cable.

In a variation B of the present embodiment, the conductors comprise the same type and size of conductor. In one example of the present variation, the conductors are 42 AWG conductors. In another example of the present variation, the conductors are 44 AWG conductors.

In a variation C of the present embodiment, at least some of the wires are coaxial and the cable is devoid of an electrical shield that surrounds the bundle of wires.

Variations of the present embodiment may be combined to form additional variations of the embodiment. Thus, variation A may be combined in a new variation with variation B. Variations A and B may be combined with variation C in a new variation.

In the embodiments of the bundles described in the preceeding two paragraphs, e.g. bundles 300, 310, 320 and 340, the data conductor and the clock conductor are arranged cross-sectionally with the data conductor positioned between two of the at least four unpaired conductors, the two of the at least four unpaired conductors not including the clock conductor. In other words, the power or ground conductors are positioned next to the data conductor, and the clock conductor is positioned next to the power or ground conductors but separated from or opposite the data conductor. Clockwise, the conductors may be arranged in the following orders: (A) data, ground, clock, and power, (B) data, power, clock, and ground, (C) data, ground, clock, camera power, and LED power, (D) data, ground, clock, LED power, and camera power, (E) data, camera power, LED power, clock, and ground, or (F) data, LED power, camera power, clock, and ground. If more conductors are present, they may be positioned in between any conductors in the foregoing orders. The conductors, without the insulation, may comprise a diameter greater than 0.070 mm and smaller than 0.220 mm. The conductors, with the insulation, may comprise a diameter greater than 0.140 mm and smaller than 0.280 mm. Separation of the data and clock wires/conductors has resulted in sufficient electrical noise mitigation from cross-talk from the clock signal. The data cable may be used to transfer an analog out signal from the camera and control signals between the camera and the VPA. The analog out signal includes the video frames and images. The control signals may be provided via a serial peripheral interface (SPI) and multiplexed with the analog signals and may include gain and exposure camera settings.

In the embodiments of the bundles described in the preceeding three paragraphs, e.g. bundle 300 and bundle 320, the bundle of at least four unpaired conductors may be enclosed in an electrical shield. The electrical shield may be electrically disconnected at the distal end of the bundle. The bundle has a proximal end. The electrical shield may only be grounded at the proximal end of the bundle. The electrical shield may be comprised by a foil or braid. Other types of electrical shields may be used as well.

The uninsulated wires range in size (e.g. diameter) from 32 AWG to 44 AWG. As is well known, AWG denotes the American Wire Gauge standard. Dimensions of the wires are given in ASTM standard B 258. The AWG tables are for a single, solid and round conductor. The AWG of a stranded wire is determined by the cross-sectional area of the equivalent solid conductor. Because there are also small gaps between the strands, a stranded wire will always have a slightly larger overall diameter than a solid wire with the same AWG. 36 AWG wire, for example, has an outer diameter of 0.127 mm. 32 AWG wire has an outer diameter of 0.202 mm. 40 AWG wire has an outer diameter of 0.080 mm. 42 AWG wire has an outer diameter of 0.063 mm. 44 AWG wire has an outer diameter of 0.050 mm. 46 AWG wire has an outer diameter of 0.040 mm. In one example, the 36 AWG wire insulation thickness is 0.06 mm, bringing the total outer diameter of the insulated wire to 0.25 mm.

In some embodiments, the bundle of conductors comprises eight conductors, as shown in the cable 400 depicted in FIGS. 19 and 20. Each of the conductors 402 is surrounded by an insulation 404 and the bundle is surrounded by a jacket 410 without an electrical shield. The conductors, depicted as A1-A8 in FIG. 20, are ordered with the A8 conductor in the center of the bundle. The conductors may be as follows. The function names are based on a serial camera control bus (SCCB) interface:

| CONDUCTOR | LABEL | FUNCTION |
|---|---|---|
| A1 | VLED | LED power |
| A2 | SDA | SCCB data |
| A3 | SCL | Input clock |
| A4 | 3V3 | Camera power |
| A5 | GND | Ground |
| A6 | CLK | Clock |
| A7 | GND | Analog circuit ground |
| A8 | Vout | Analog output |

In one embodiment, the A8 conductor is the Vₒᵤₜ conductor.

In some embodiments, at least four of the wires comprise two twisted pairs of wires. The twisted pairs may comprise Vout and ground, and clock and ground. The twisted pair including Vout is positioned in the perimeter of the bundle, not in the center. Additional wires may be twisted or untwisted. In one example, four of eight wires are untwisted.

Generally, all the wires are at most 34 AWG wires in size, and may be 36, 38, 40 42 or 46 AWG wires. Smaller gauge wires can aid in reducing the cross-sectional size of the intrusive portion of the intrusive medical device and increasing bending flexibility but at the expense of noise sensitivity. When size reduction is not needed heavier gauge wires may be preferrable.

Although some embodiments have been described and shown in detail, the invention is not restricted to them but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same hardware components. The mere fact that certain measures are recited in mutually different dependent items or described in different embodiments does not indicate that a combination of these measures cannot be used to advantage.

The terms "first," "second," "third," "fourth," and the like in the description and in the claims, if any, are used for distinguishing between similar elements and not necessarily for describing a particular sequential or chronological order. It is to be understood that any terms so used are interchangeable under appropriate circumstances such that the embodiments described herein are, for example, capable of operation in sequences other than those illustrated or otherwise described herein.

It should be emphasized that the term "comprises/comprising" are generally interpreted to be open ended terms which specify the presence of stated features, integers, steps or components but do not preclude the presence or addition of one or more other features, integers, steps, components or groups thereof. The terms "consisting of" or "consists of" are closed terms, and include only the components, structures, steps, or the like specifically listed in conjunction with such terms, as well as that which is in accordance with U.S. Patent law.

### Reference numerals:

| REFERENCE NUMERALS: | |
|---|---|
| 20 | Visualization system |
| 30, 240, 260 | Video processing apparatus (VPA) |
| 32 | Cable connector receptor |
| 34 | Input circuit |
| 36 | Processor |
| 40 | intrusive medical device |
| 40p | Proximal end |
| 40d | Distal end |
| 42 | Cable |
| 44 | Cable connector |
| 46 | Positioning interface |
| 48 | Circuit board |
| 50 | Tubular member |
| 50p | Proximal end |
| 50d | Distal end |
| 52 | Tubular member wall |
| 54 | lumen |
| 56 | Proximal end |
| 58 | Distal end |
| 60 | Camera |
| 62 | Circuit board |
| 64 | Light emitting diode (LED) |
| 70 | Bundle of conductors |
| 71 | filler |
| 72 | Conductors |
| 74 | Electrical shield |
| 76 | proximal-end shield ground |
| 80 | electrosurgery system |
| 82 | Electrode |
| 84 | Electrode |
| 86 | Electrical path |
| 90 | Display screen |
| 92 | Display screen support |
| 140 | Endoscope |
| 146 | Handle |
| 148 | Steering control |
| 150 | Insertion cord |
| 150d | Distal end |
| 150p | Proximal end |
| 152 | Insertion tube |
| 160, 190, 192, 194 | Bending section |
| 162 | Proximal segment |
| 164 | Distal segment |
| 166 | Segment |
| 168 | Hinge |
| 170 | Tip housing |
| 172 | Working channel tube |
| 174 | Steering cable |
| 176 | steering cable guide tubes |
| 176' | apertures |
| 180 | Common opening |
| 180' | Common opening |
| 182 | Working channel tube opening |
| 184 | steering cable Cut-out |
| 186 | bundle Cut-out |
| 192 | Bending section |
| 194 | Bending section |
| 200 | Dual-lumen tube |
| 200a | Tubular member |
| 201 | Circumferential wall |
| 202 | First lumen |
| 204 | Second lumen |
| 206 | LED |
| 208 | Illumination lumen |
| 210 | first portion of wall |
| 212 | second portion of wall |
| 214 | Medial wall |
| 220 | Cuff |
| 222 | Inflation tube |
| 242, 262 | Housing |
| 244 | Display screen |
| 246 | Cable connector receptor/receptacle |
| 300, 310, 320, 340 | bundle |
| 304, 326, 404 | Insulator/insulation |
| 306, 330, 410 | jacket |
| 322, 324, 326 | Wire |
| 328 | Electrical shield |
| 332 | Binder tape |
| 400 | Cable |
| 402 | Conductor |
| P | Pitch |
| EST | Electro-surgical tool |

## Claims

1. An intrusive medical device (40, 140, 200) comprising:
a proximal end (40p) and a distal end (40d) spaced apart from the proximal end;
a tubular member (50) defining a lumen (54) therein, the tubular member extending from the proximal end to the distal end;
a camera (60) positioned at the distal end; and
a bundle (70) of at least four unpaired conductors, the bundle extending from the proximal end to the distal end and electrically connected to the camera at the distal end,
wherein the bundle is twisted at a pitch of 15 mm +/- 10 mm.

2. The intrusive medical device (40, 140, 200) of claim 1, wherein the at least four unpaired conductors include a ground conductor, a camera power conductor, a clock conductor and a data conductor, wherein the at least four unpaired conductors are arranged cross-sectionally with the data conductor positioned between two of the at least four unpaired conductors, and wherein the two of the at least four unpaired conductors does not include the clock conductor.

3. The intrusive medical device (40, 140, 200) of claim 2, wherein the at least four unpaired conductors consist of five unpaired conductors including the ground conductor, the camera power conductor, the clock conductor, the data conductor, and a light power conductor, and wherein the data conductor is positioned immediately next to the ground conductor and/or the camera power conductor and/or the light power conductor.

4. The intrusive medical device (40, 140, 200) of any of the preceding claims, wherein each of the conductors is insulated, and wherein each of the conductors, without the insulation, comprises a diameter of between 0.0790 and 0.210 mm.

5. The intrusive medical device (40, 140, 200) of any of the preceding claims, wherein at least three of the at least four conductors are coaxial.

6. The intrusive medical device (40, 140) of any one of the preceding claims, wherein the intrusive medical device comprises an endoscope (140) including:
a positioning interface (146) having a distal end;
an insertion cord (150) connected to and extending from the distal end of the positioning interface and comprising an insertion tube (152), a bending section (160), and a tip housing (170), the camera positioned in the tip housing, the tubular member extending from the positioning interface through the insertion tube to the tip housing,
wherein the bundle extends from the positioning interface to the tip housing.

7. The intrusive medical device (40, 140) of claim 6, wherein the bundle of at least four unpaired conductors is enclosed in an electrical shield (74).

8. The intrusive medical device (40, 140) of claim 7, wherein the electrical shield is electrically disconnected at the distal end of the bundle.

9. The intrusive medical device (40, 140) of any one of claims 6 to 8, wherein the tip housing comprises a diameter smaller than 3.4 mm.

10. The intrusive medical device (40, 140) of claim 6, wherein the tip housing comprises a diameter smaller than 3.4 mm, wherein the bundle of at least four unpaired conductors is enclosed in an electrical shield, and wherein the electrical shield is electrically disconnected at the distal end of the bundle.

11. The intrusive medical device (40, 200) of claim 1, further comprising:
a tubular member (200a) having a peripheral wall (201) defining a lumen (202) therein; and
an illumination lumen (208) within the peripheral wall,
wherein the camera and the bundle of at least four unpaired conductors are positioned in the illumination lumen.

12. The intrusive medical device (40, 200) of any of claim 11, wherein the at least four unpaired conductors include a ground conductor, a camera power conductor, a clock conductor and a data conductor, wherein the at least four unpaired conductors are arranged cross-sectionally with the data conductor positioned between two of the at least four unpaired conductors, and wherein the two of the at least four unpaired conductors does not include the clock conductor.

13. The intrusive medical device (40, 200) of claim 12, wherein the at least four unpaired conductors consist of five unpaired conductors including the ground conductor, the camera power conductor, the clock conductor, the data conductor, and a light power conductor, and wherein the data conductor is positioned immediately next to the ground conductor and/or the camera power conductor and/or the light power conductor.

14. The intrusive medical device (40, 200) of claim 11, wherein the at least four unpaired conductors include eight conductors including a video out conductor positioned in a center of the bundle and surrounded by the other of the eight conductors.

15. A visualization system (20) comprising:
the intrusive medical device (40, 140, 200) of any of the preceding claims; and
a video processing apparatus (30) configured to communicatively connect with the intrusive medical device to receive a video stream therefrom.

## Patentansprüche

1. Intrusive medizinische Vorrichtung (40, 140, 200), umfassend:
ein proximales Ende (40p) und ein distales Ende (40d), das von dem proximalen Ende beabstandet ist;
ein röhrenförmiges Element (50), das ein Lumen (54) darin definiert, wobei sich das röhrenförmige Element von dem proximalen Ende zu dem distalen Ende erstreckt;
eine Kamera (60), die an dem distalen Ende positioniert ist; und
ein Bündel (70) aus wenigstens vier ungepaarten Leitern, wobei sich das Bündel von dem proximalen Ende zu dem distalen Ende erstreckt und elektrisch mit der Kamera am distalen Ende verbunden ist,
wobei das Bündel mit einer Steigung von 15 mm +/- 10 mm verdrillt ist.

2. Intrusive medizinische Vorrichtung (40, 140, 200) nach Anspruch 1, wobei die wenigstens vier ungepaarten Leiter einen Masseleiter, einen Kamerastromleiter, einen Taktleiter und einen Datenleiter beinhalten, wobei die wenigstens vier ungepaarten Leiter querschnittlich angeordnet sind, wobei der Datenleiter zwischen zwei der wenigstens vier ungepaarten Leiter positioniert ist, und wobei die zwei der wenigstens vier ungepaarten Leiter nicht den Taktleiter beinhalten.

3. Intrusive medizinische Vorrichtung (40, 140, 200) nach Anspruch 2, wobei die wenigstens vier ungepaarten Leiter aus fünf ungepaarten Leitern bestehen, beinhaltend den Masseleiter, den Kamerastromleiter, den Taktleiter, den Datenleiter und einen Lichtstromleiter, und wobei der Datenleiter unmittelbar neben dem Masseleiter und/oder dem Kamerastromleiter und/oder dem Lichtstromleiter positioniert ist.

4. Intrusive medizinische Vorrichtung (40, 140, 200) nach einem der vorstehenden Ansprüche, wobei jeder der Leiter isoliert ist und wobei jeder Leiter, ohne die Isolierung, einen Durchmesser zwischen 0,0790 und 0,210 mm umfasst.

5. Intrusive medizinische Vorrichtung (40, 140, 200) nach einem der vorstehenden Ansprüche, wobei wenigstens drei der wenigstens vier Leiter koaxial sind.

6. Intrusive medizinische Vorrichtung (40, 140) nach einem der vorstehenden Ansprüche, wobei die intrusive medizinische Vorrichtung ein Endoskop (140) umfasst, aufweisend:
eine Positionierungsschnittstelle (146) mit einem distalen Ende;
ein Einführungskabel (150), das mit der Positionierungsschnittstelle verbunden ist und sich vom distalen Ende derselben erstreckt und einen Einführtubus (152), einen Biegeabschnitt (160) und ein Spitzengehäuse (170) umfasst, wobei die Kamera in dem Spitzengehäuse positioniert ist, das röhrenförmiges Element sich von der Positionierungsschnittstelle durch den Einführtubus zu dem Spitzengehäuse erstreckt,
wobei sich das Bündel von der Positionierungsschnittstelle zu dem Spitzengehäuse erstreckt.

7. Intrusive medizinische Vorrichtung (40, 140) nach Anspruch 6, wobei das Bündel aus wenigstens vier ungepaarten Leitern in einer elektrischen Abschirmung (74) eingeschlossen ist.

8. Intrusive medizinische Vorrichtung (40, 140) nach Anspruch 7, wobei die elektrische Abschirmung am distalen Ende des Bündels elektrisch getrennt ist.

9. Intrusive medizinische Vorrichtung (40, 140) nach einem der Ansprüche 6 bis 8, wobei das Spitzengehäuse einen Durchmesser kleiner als 3,4 mm umfasst.

10. Intrusive medizinische Vorrichtung (40, 140) nach Anspruch 6, wobei das Spitzengehäuse einen Durchmesser kleiner als 3,4 mm umfasst, wobei das Bündel aus wenigstens vier ungepaarten Leitern in einer elektrischen Abschirmung eingeschlossen ist und wobei die elektrische Abschirmung am distalen Ende des Bündels elektrisch getrennt ist.

11. Intrusive medizinische Vorrichtung (40, 200) nach Anspruch 1, ferner umfassend:
ein röhrenförmiges Element (200a) mit einer peripheren Wand (201), die ein Lumen (202) darin definiert; und
ein Beleuchtungslumen (208) in der peripheren Wand,
wobei die Kamera und das Bündel aus wenigstens vier ungepaarten Leitern in dem Beleuchtungslumen positioniert sind.

12. Intrusive medizinische Vorrichtung (40, 200) nach Anspruch 11, wobei die wenigstens vier ungepaarten Leiter einen Masseleiter, einen Kamerastromleiter, einen Taktleiter und einen Datenleiter aufweisen, wobei die wenigstens vier ungepaarten Leiter querschnittlich angeordnet sind, wobei der Datenleiter zwischen zwei der wenigstens vier ungepaarten Leiter positioniert ist, und wobei die zwei der wenigstens vier ungepaarten Leiter den Taktleiter nicht einschließen.

13. Intrusive medizinische Vorrichtung (40, 200) nach Anspruch 12, wobei die wenigstens vier ungepaarten Leiter aus fünf ungepaarten Leitern bestehen, beinhaltend den Masseleiter, den Kamerastromleiter, den Taktleiter, den Datenleiter und einen Lichtstromleiter, und wobei der Datenleiter unmittelbar neben dem Masseleiter und/oder dem Kamerastromleiter und/oder dem Lichtstromleiter positioniert ist.

14. Intrusive medizinische Vorrichtung (40, 200) nach Anspruch 11, wobei die wenigstens vier ungepaarten Leiter acht Leiter aufweisen, beinhaltend einen Videoausgangsleiter, der in einer Mitte des Bündels positioniert ist und von den anderen der acht Leiter umgeben ist.

15. Visualisierungssystem (20), umfassend:
die intrusive medizinische Vorrichtung (40, 140, 200) nach einem der vorstehenden Ansprüche; und
eine Videoverarbeitungseinrichtung (30), die dazu ausgelegt ist, kommunikativ mit der intrusiven medizinischen Vorrichtung verbunden zu werden, um einen Videostrom von dieser zu empfangen.

## Revendications

1. Dispositif médical intrusif (40, 140, 200) comprenant :
une extrémité proximale (40p) et une extrémité distale (40d) espacée de l'extrémité proximale ;
un élément tubulaire (50) définissant une lumière (54) à l'intérieur, l'élément tubulaire s'étendant de l'extrémité proximale à l'extrémité distale ;
une caméra (60) positionnée à l'extrémité distale ; et
un faisceau (70) d'au moins quatre conducteurs non appariés, le faisceau s'étendant depuis l'extrémité proximale jusqu'à l'extrémité distale et étant connecté électriquement à la caméra à l'extrémité distale,
le faisceau étant torsadé selon un pas de 15 mm +/- 10 mm.

2. Dispositif médical intrusif (40, 140, 200) selon la revendication 1, les au moins quatre conducteurs non appariés comprenant un conducteur de terre, un conducteur d'alimentation de caméra, un conducteur d'horloge et un conducteur de données, les au moins quatre conducteurs non appariés étant agencés en section transversale avec le conducteur de données positionné entre deux des au moins quatre conducteurs non appariés, et les deux des au moins quatre conducteurs non appariés ne comprenant pas le conducteur d'horloge.

3. Dispositif médical intrusif (40, 140, 200) selon la revendication 2, les au moins quatre conducteurs non appariés étant constitués de cinq conducteurs non appariés comprenant le conducteur de terre, le conducteur d'alimentation de caméra, le conducteur d'horloge, le conducteur de données et un conducteur d'alimentation de lumière, et le conducteur de données étant positionné immédiatement à côté du conducteur de terre et/ou du conducteur d'alimentation de caméra et/ou du conducteur d'alimentation de lumière.

4. Dispositif médical intrusif (40, 140, 200) selon l'une quelconque des revendications précédentes, chacun des conducteurs étant isolé, et chacun des conducteurs, sans l'isolation, ayant un diamètre compris entre 0,0790 et 0,210 mm.

5. Dispositif médical intrusif (40, 140, 200) selon l'une quelconque des revendications précédentes, au moins trois des au moins quatre conducteurs étant coaxiaux.

6. Dispositif médical intrusif (40, 140) selon l'une quelconque des revendications précédentes, le dispositif médical intrusif comprenant un endoscope (140) comprenant :
une interface de positionnement (146) ayant une extrémité distale ;
un cordon d'insertion (150) connecté à l'extrémité distale de l'interface de positionnement et s'étendant à partir de celle-ci, et comprenant un tube d'insertion (152), une section de flexion (160) et un boîtier d'extrémité (170), la caméra étant positionnée dans le boîtier d'extrémité, l'élément tubulaire s'étendant depuis l'interface de positionnement à travers le tube d'insertion jusqu'au boîtier d'extrémité,
le faisceau s'étendant depuis l'interface de positionnement jusqu'au boîtier d'extrémité.

7. Dispositif médical intrusif (40, 140) selon la revendication 6, le faisceau d'au moins quatre conducteurs non appariés étant enfermé dans un blindage électrique (74).

8. Dispositif médical intrusif (40, 140) selon la revendication 7, le blindage électrique étant déconnecté électriquement à l'extrémité distale du faisceau.

9. Dispositif médical intrusif (40, 140) selon l'une quelconque des revendications 6 à 8, le boîtier d'extrémité ayant un diamètre inférieur à 3,4 mm.

10. Dispositif médical intrusif (40, 140) selon la revendication 6, le boîtier d'extrémité comprenant un diamètre inférieur à 3,4 mm, le faisceau d'au moins quatre conducteurs non appariés étant enfermé dans un blindage électrique, et le blindage électrique étant déconnecté électriquement à l'extrémité distale du faisceau.

11. Dispositif médical intrusif (40, 200) selon la revendication 1, comprenant en outre :
un élément tubulaire (200a) ayant une paroi périphérique (201) définissant une lumière (202) à l'intérieur ; et
une lumière d'éclairage (208) à l'intérieur de la paroi périphérique,
la caméra et le faisceau d'au moins quatre conducteurs non appariés étant positionnés dans la lumière d'éclairage.

12. Dispositif médical intrusif (40, 200) selon l'une quelconque des revendications 11, les au moins quatre conducteurs non appariés comprenant un conducteur de terre, un conducteur d'alimentation de caméra, un conducteur d'horloge et un conducteur de données, les au moins quatre conducteurs non appariés étant agencés en section transversale avec le conducteur de données positionné entre deux des au moins quatre conducteurs non appariés, et les deux des au moins quatre conducteurs non appariés ne comprenant pas le conducteur d'horloge.

13. Dispositif médical intrusif (40, 200) selon la revendication 12, les au moins quatre conducteurs non appariés étant constitués de cinq conducteurs non appariés comprenant le conducteur de terre, le conducteur d'alimentation de caméra, le conducteur d'horloge, le conducteur de données et un conducteur d'alimentation de lumière, et le conducteur de données étant positionné immédiatement à côté du conducteur de terre et/ou du conducteur d'alimentation de caméra et/ou du conducteur d'alimentation de lumière.

14. Dispositif médical intrusif (40, 200) selon la revendication 11, les au moins quatre conducteurs non appariés comprenant huit conducteurs comprenant un conducteur de sortie vidéo positionné au centre du faisceau et entouré par les autres des huit conducteurs.

15. Système de visualisation (20) comprenant :
le dispositif médical intrusif (40, 140, 200) selon l'une quelconque des revendications précédentes ; et
un appareil de traitement vidéo (30) configuré pour se connecter de manière communicative avec le dispositif médical intrusif afin de recevoir un flux vidéo provenant de celui-ci.
